Europäisches Patentamt

(19) European Patent Office

Office européen des brevets

(11) Publication number : **0 176 585**
**B1**

(12) EUROPEAN PATENT SPECIFICATION

(45) Date of publication of patent specification :
27.07.88

(51) Int. Cl.⁴ : **C 12 Q 1/46**, C 12 N 11/12

(21) Application number : 85902180.0

(22) Date of filing : 02.04.85

(86) International application number :
PCT/SE 85/00157

(87) International publication number :
WO/8504423 (10.10.85 Gazette 85/22)

(54) ENZYME PAPER FOR THE INDICATION OF CHOLINESTERASE-INHIBITORS, THE PREPARATION AND USE THEREOF.

(30) Priority : 02.04.84 SE 8401797

(43) Date of publication of application :
09.04.86 Bulletin 86/15

(45) Publication of the grant of the patent :
27.07.88 Bulletin 88/30

(84) Designated contracting states :
AT BE CH DE FR GB IT LI LU NL SE

(56) References cited :
US-A- 3 809 617
US-A- 4 241 180
US-A- 4 324 858
Chemical Abstracts Vol 84, abstract 146930j published 1976, Anal. Biochem. 1976, 71(1), 273-80

(73) Proprietor : DAHLGREN, Erik
Marmorvägen 127
S-902 42 Umea (SE)

BERGEK, Sture
Björnvägen 46
S-902 60 Umea (SE)

MARTENSSON, Kaj
Rondovägen 12
S-245 00 Staffanstorp (SE)

(72) Inventor : DAHLGREN, Erik
Marmorvägen 127
S-902 42 Umea (SE)
Inventor : BERGEK, Sture
Björnvägen 46
S-902 60 Umea (SE)
Inventor : MARTENSSON, Kaj
Rondovägen 12
S-245 00 Staffanstorp (SE)

(74) Representative : Robinson, Marianne et al
Försvarets civilförvaltning Patent Section Box 80012
Östermalmsgatan 87
S-104 50 Stockholm (SE)

## Description

The present invention relates to an enzyme paper for use in the indication of cholinesterase-inhibitors in for instance air or water, the paper exhibiting among other things enhanced sensitivity, storage stability and wettability, and a method of making the enzyme paper. The enzyme paper is suitable for indication of nerve gases or other organophosphorous compounds of cholinesterase-inhibiting nature, such as plant control means, e. g. weed-killers, and plant protectants.

It is previously known to use paper impregnated with an enzyme of the cholinesterase type in equipments for indication of nerve gases. Nerve gases inactivate enzymes of this type, which can be illustrated by a colouring reaction normally developed by the enzyme in contact with a suitable substrate. The presence of nerve gas is indicated by the fact that the colour change of the substrate, which is normally developed by the enzyme, fails to come off because of the cholinesterase-inhibiting function of the nerve gas. In the U.S. patent No 3 049 411 is thus described the use of a filter paper preimpregnated with the enzyme cholinesterase, which through hydrolysis decomposes a substrate such as 2,6-dichloro indophenyl acetate or indoxyl acetate with a colour change to blue as the result. The filter paper, which has been pretreated with cholinesterase and buffered to a pH of 6.4-8.5, is moistened and exposed to the atmosphere that is to be tested and then brought into contact with the substrate in an aqueous solution buffered to a pH of about 6.4-8.5. When exposed to air comprising nerve gas, the enzyme is phosphorylated, and in such a case the blue colouring will not occur during the subsequent contact with the substrate.

However, it has been found that problems exist with known enzyme papers, which have been preimpregnated with the enzyme and dried for use later on, namely problems with the stability of the paper during storage and the wettability of the paper when being moistened before the exposure. According to the Swedish patent No 314 041 attempts have been made to use enzyme paper, which has not been moistened. Cholinesterase, however, has the character of a glue, and a dried impregnation layer can only slowly be influenced by the sample air.

Another problem with enzyme papers of this kind is that the enzyme is easily dissolved out of the paper when in contact with water, which makes the use of the enzyme paper impossible for the indication of cholinesterase-inhibitors in water. For the same reason the coloured substrate paper will often become unevenly coloured, also in air indication, which may give rise to unclear evaluations of the indication result, as the enzyme paper must be moistened before use in order to function satisfactory.

In the U.S. patent No 4 324 858 a cholinesterase enzyme, preferably from electric eel or horse serum, is stabilized on paper by the use of a zwitterionic buffer and extreme drying of the impregnated enzyme paper.

A critical factor is of course the enzyme sensitivity to low concentrations of cholinesterase-inhibitors. Existing enzyme papers have a not totally satisfying lower detection limit to for instance the nerve gas VX. This limit is above all dependent on the choice of enzyme, where cholinesterase from plaice has expecially good sensitivity qualities compared with traditionally used enzyme sources of the type bovine serum or electric eel or ray. The plaice enzyme has, however, normally not completely satisfactory stability qualities, which makes the use thereof difficult on conventional enzyme papers.

A possible technique to overcome some of the above problems is to immobilize the enzyme to the paper, i. e. to bind the enzyme chemically or physically to the carrier material, covalently, electrostatically or by means of hydrophobic interaction. This technique is previously known for other enzymes but not for cholinesterase from plaice.

It is known to a man skilled in the art that immobilization of an enzyme normally means a substantial loss of enzyme activity and far from always results in a stabilization olf the enzyme. This is evident for instance from table 5. It is neither to be expected that the sensitivity qualities of the enzyme will be unchanged. Sooner is an impaired sensitivity to be expected, partly because of occurring steric hindrances in the micro-environment of the enzyme, partly because of the fact that a large quantity of the enzyme is normally inactivated from the immobilization resulting in a lower specific activity. Usually an enzyme immobilization also means a substantial extra cost for the production of the end product.

A wetting agent is often used in other connections in order to improve the wettability of products. Most wetting agents, however, have normally an inactivating and/or destabilizing effect on the activity of enzymes. Besides, an unsuitable choice of wetting agent can make the immobilization of the enzyme more difficult.

The present invention relates to an enzyme paper, where the enzyme cholinesterase from plaice is immobilized to an ion exchange paper by means of electrostatic adsorption. This gives a product with unique qualities, which are unexpectedly much better in several aspects compared to what a man skilled in the art could expect. What is unique is above all that the combination of all good qualities of the above-mentioned kind can be obtained at the same time on one and the same paper. This combination of good qualities is not previously known, and the enzyme paper is decidedly superior to the corresponding products known up to now.

The obtained combination of positive qualities is summarized as follows :

0 176 585

a) The product can without any problems be used for both air indication and water indication.

b) The enzyme paper gives an evenly coloured surface of hydrolyzed substrate on the substrate paper, i. e. a distinct indication that cannot be misinterpreted.

c) The sensitivity qualities are better than the qualities of the corresponding existing products.

d) The wettability of the paper is correspondingly considerably better compared with previously known enzyme papers.

e) The stability qualities of the enzyme are improved considerably in relation to free, watersoluble cholinesterase from plaice, which makes the stability of the product better than most of the corresponding enzyme papers existing on the market.

f) A very small loss of enzyme activity occurs because of the immobilization method used, which is essential from sensitivity point of view as well as from cost point of view.

g) The technique, which is used in the production of the enzyme paper, is very simple, which means that the production cost can be kept sufficiently low.

The characteristics of the invention are evident from the subsequent patent claims.

By using, in the production of the enzyme paper, a paper of the type chromatography paper with ion exchange function, preferably with anion exchange function and good mechanical wet strength, in combination with an enzyme solution comprising, beside the enzyme, a surface-active agent and a stabilizing component of the carbohydrate type, there is obtained an unchanged or insignificantly reduced enzyme activity in the enzyme paper. The enzyme is hereby immobilized at the same time as an unexpectedly high coupling yield of immobilized enzyme is obtained and also an unexpectedly high specific activity of the immobilized enzyme. This is very unexpected, as immobilization methods, as has already been pointed out, normally lead to considerably impaired enzyme activity qualities (see table 5). The sensitivity qualities of the immobilized enzyme are unchanged at the same time as the wettability of the enzyme paper is greatly improved. It is very unexpected that the wettability is improved without being accompanied by a decrease in enzyme activity, enzyme stability, coupling yield or sensitivity, as a wetting agent normally inactivates enzymes to a greater or less extent. Because of the choice of immobilization method and the addition of the stabilizing component, an unexpectedly good enzyme stability of the finished product is achieved. Most other such combinations will not give any improvement but often result in a destabilization as well.

In a specially suitable embodiment of the invention a chromatography paper of DEAE-cellulose is used of the type Whatman DE 81, and as a wetting agent there is used a nonionic agent, e. g. Tween 80® (Polysorbate 80). The enzyme is preferably cholinesterase from plaice, and as stabilizing components are preferably used saccharose and a lowmolecular dextran, e. g. dextran T 10 (Pharmacia Fine Chemicals). The enzyme solution may be buffered, preferably with a phosphate buffer.

The substrate, which has proved to have the best qualities for an enzymatic colour reaction of the described type, is 2,6-dichloro indophenyl acetate (DCIPA). The substrate is suitably applied on an absorbing paper, e. g. chromatography paper or filter paper.

The invention will now be described in more detail by means of a number of examples.

Production of enzyme paper

Example 1 : Enzyme immobilized to DEAE-cellulose paper

Chromatography paper of the type Whatman DE 81 is equilibrated with a 1 M solution of NaCl for about 30 minutes, carefully washed with distilled water and dried at room temperature. On suitably large pieces of this paper, which are cut or punched out from the paper, e. g. round discs of the diameter 20 mm, there is applied 5 $\mu$m of an enzyme solution in 0.1 M phosphate buffer (pH 7.4) consisting of cholinesterase from plaice with a specific activity of 1.46 $\mu$/mg or 0.83 $\mu$mol/min/mg (40 mg/ml), saccharose (160 mg/ml), dextran T 10 (160 mg/ml) and as a wetting agent Tween 80 (4 $\mu$l/ml). The enzyme papers thus produced are air-dried at room temperature and then dried in an exsiccator under vacuum.

Exemple 2 : Storage stability of enzyme papers

During storage the enzyme papers are suitably enclosed in a tight package material. In this test there have been used welded polypropylene bags, and acceptable storage times are evident from the following table.

Table 1

| Storage temperature | Storage time |
|---|---|
| — 30 °C | > 5 years |
| 20-25 °C | > 3 years |
| 40 °C | > 1.5 years |
| 62-68 °C | 4 months |

3

For a comparative purpose storage tests have been carried out with some alternative combinations of enzyme papers at a storage temperature of 40 °C. The enzyme papers have been produced according to example 1 (in applicable parts), and the results are evident from table 2, in which

E = plaice enzyme (0.2 mg) ; 1.46 $\mu$/mg ; 1.62 $\mu$/mg protein
S = saccharose (0.8 mg)
D = dextran T 10 (0.8 mg)
T = Tween 80 (0.02 $\mu$l)

Table 2

| Paper quality | Components | Enzyme yield (%) | The enzyme half-life (months), i.e. 50% remaining activity of initial value |
|---|---|---|---|
| DEAE-cellulose (Whatman DE 81) | E, S, D, T (according to the invention) | 59 | > 18 |
| " | E, S, D | 62 | > 18 |
| " | E, T | 20 | < 1 |
| Cellulose (Whatman 113) | E, S, D, T | 45 | ~ 5 |

From table 2 is evident that the storage stability is inferior with the filter paper type Whatman 113 than with Whatman 81, which is a paper of the type chromatography paper with ion exchange function. An immobilization of the enzyme does not occur with a paper of the type Whatman 113. The results in table 2 show that immobilization of the enzyme gives an improved storage stability. Moreover there is seen the positive effect of the stabilizing components saccharose and dextran.

Production of substrate paper

Example 3

2,6-dichloro indophenol is acetylated with a large excess of acetic anhydride to 2,6-dichloro indophenyl acetate (DCIPA), which is precipitated in water and recrystallized in n-hexane : diethyl ether.

Whatman 113 paper is impregnated with a saturated solution of DCIPA in benzine (50 $\mu$l DCIPA/cm$^2$ paper), and then the substrate paper is airdried at room temperature. Suitably large pieces of the substrate paper are punched out.

As a carrier for DCIPA the following paper types have also proved to be suitable : the chromatography paper Whatman No 1 and the filter papers Munktell OOH, OOM and IF.

Besides in benzine, DCIPA can be dissolved in other solvents, such as acetone, carbon tetrachloride, chloroform, dichloromethan or 1,2-dichloroethan, and then so much of the solution is dropwise added to the paper that the whole paper becomes evenly moistened. The surface concentration, i. e. the amount of DCIPA per cm$^2$, can be varied by changing the concentration of DCIPA in the solution. It has been found that the surface concentration is not critical as long as a sufficiently large amount of substrate is available in order to give a clear change in the enzymatic colour reaction. Preferably there is used a relatively high surface concentration, e. g. 80-100 $\mu$g/cm$^2$, in order to compensate the losses in the form of decomposition and possibly sublimation of the substrate that has to be accounted for during storage.

In order to get an improved stability of DCIPA during storage for a longer period of time of the substrate paper produced as described above, it should be stored together with a desiccant, e. g. silica gel or molecular sieve (4 A).

# 0 176 585

## Indication

### Example 4 : Indication of cholinesterase-inhibitor in gas

An enzyme paper, produced as described in example 1, is moistened with about 4 drops of distilled water, and then the paper is exposed for 2 minutes to the gas, which is suspected to comprise or consist of cholinesterase-inhibitors. The paper is developed by pressing a substrate paper towards it for 2 minutes, and then the result is evaluated. An uncoloured, pink or very faint grey colour indicates that cholinesterase-inhibitors are present in the tested gas, while a blue or faint blue colouring indicates that no cholinesterase-inhibitors are present. The enzyme paper sensitivity in the indication of sarin, i. e. isopropylmethyl phosphono fluoridate, and other organo-phosphorous compounds that summarized are named nerve gases, is evident from the following table.

#### Table 3

| Cholinesterase-inhibitor | Paper sensitivity (mg/m$^3$) |
|---|---|
| Sarin | 0.04 |
| VX | 0.04 |

### Example 5 : Indication of cholinesterase-inhibitor in liquid

Enzyme paper produced according to example 1 is, without being initially moistened, dipped during stirring into an aqueous solution comprising the actual inhibitor for 2 minutes. The development of the paper is carried out as described in example 4. The enzyme paper sensitivity is evident from table 4.

#### Table 4

| Inhibitor | Paper sensitivity (mg/l) |
|---|---|
| Sarin | 0.6 |
| VX | 0.05 |

## Comparative tests

### Example 6

Other methods of immobilizing the enzyme to a carrier were carried out for a comparative purpose. It was found that unexpectedly good results were obtained with the method according to the invention from coupling yield point of view (about 60 %) compared with other techniques (about 0-7 %), which can be seen from the following table.

#### Table 5

| Immobilizing method | Enzyme activity (u) before immobiliza-tion | Activity of immobilized preparation | |
|---|---|---|---|
| | | u | % |
| Adsorption to | | | |
| Amberlite XAD 7 | 29.2 | 0.2 | 0.7 |
| XAD 2 | 29.2 | 0 | 0 |
| IR 120-Al$^{3+}$ | 29.2 | 0.1 | 0.3 |
| Adsorption to | | | |
| Hexylsepharose 6 B | 29.2 | 0.9 | 3.1 |
| Hexylsepharose 6 B + substrate | 29.2 | 0.6 | 2.1 |
| Hexylcellulose | 29.2 | 0.5 | 1.7 |
| Whatman 1 PS | 29.2 | 0 | 0 |
| Whatman 1 PS + $C_{12}H_{25}OSO_3NA$ | 29.2 | 0 | 0 |

5

Table 5 (Continued)

| Immobilizing method | Enzyme activity (u) before immobiliza-tion | Activity of immobilized preparation | |
|---|---|---|---|
| | | u | % |
| Adsorption with special technique to | | | |
| Whatman SG 81 | 5.84 | 0.4 | 6.8 |
| Whatman 4 CHR | 5.84 | 0.2 | 3.4 |
| Crosslinking with glu-taraldehyde to | | | |
| Whatman SG 81 | 58.4 | 1.6 | 2.7 |
| Whatman GF/B | 58.4 | 0.8 | 1.4 |
| Crosslinking with glu-taraldehyde, special technique, to | | | |
| Whatman SG 81 | 5.84 | 0.13 | 2.2 |
| Whatman 4 CHR | 5.84 | 0.06 | 1.0 |
| Covalent bonding by means of 2-amino-4,6-dichloro triazine to | | | |
| Whatman 4 CHR | 29.2 | 0.1 | 0.3 |
| Whatman 4 CHR + substrate | 29.2 | 0.3 | 1.0 |
| Covalent bonding by means of CNBr to | | | |
| Whatman 4 CHR | 29.2 | 0.3 | 1.0 |
| Whatman 4 CHR + substrate | 29.2 | 0.4 | 1.4 |
| Covalent immobiliza-tion by means of glu-taraldehyde to | | | |
| Whatman GF/C | 29.2 | 0.3 | 1.0 |
| Whatman GF/C + substrate | 29.2 | 0.3 | 1.0 |

6

Table 5 (Continued)

| Immobilizing method | Enzyme activity (u) before immobiliza-tion | Activity of immobilized preparation | |
|---|---|---|---|
| | | u | % |
| Covalent bonding by means of carbodiimide to | ··· | | |
| Whatman GF/C | 23.0 | 0.62 | 2.7 |
| Whatman GF/C + substrate | 23.0 | 0.40 | 1.7 |
| Whatman CM 82 | 23.0 | 0.47 | 2.0 |
| Whatman DM 82 + substrate | 23.0 | 0.56 | 2.4 |
| Adsorption to | | | |
| Whatman DE 81 + saccharose and dextran T 10 | 29.2 | 17.5 | 60 |
| Whatman DE 81 + saccharose and dextran | 29.2 | 16.5 | 57 |
| Whatman DE 81 + saccharose, dextran and Tween 80 | 29.2 | 17.2 | 59 |
| Whatman DE 81 + saccharose and dextran | 29.2 | 18.1 | 62 |

## Example 7

Enzyme paper was produced according to example 1 but also without the addition of a wetting agent, and then the indication ability, i. e. the enzyme paper sensitivity, was evaluated. From table 6 is seen the indication threshold in the indication of cholinesterase-inhibitors in gas and liquid on paper with or without a wetting agent (Tween 80).

Table 6

| Cholinesterase-inhibitor | Indication threshold | |
|---|---|---|
| | gas (mg/m$^3$) | liquid (mg/l) |
| Sarin; with wetting agent | 0.04 | 0.6 |
| VX; with wetting agent | 0.04 | 0.05 |
| Sarin; without wetting agent | 0.04 | 0.6 |
| VX; without wetting agent | 0.04 | 0.05 |

From the table is evident that the addition of the wetting agent does not impair the enzyme paper sensitivity.

Tests show that neither is the stability impaired from the wetting agent addition (see table 7). With relative activity is meant enzyme activity in % of the activity at the time 0 months.

Table 7

|  | Storage temp. | Storage time | Rel.act. (%) |
|---|---|---|---|
| With Tween 80 | 40°C | 5 months | 68, 88 |
| Without Tween 80 | 40°C | 5 months | 67 |
| With Tween 80 | 62-68°C | 0.5 months | 60, 71 |
| Without Tween 80 | 62-68°C | 0.5 months | 65 |
| With Tween 80 | 62-68°C | 5.5 months | 27, 20 |
| Without Tween 80 | 62-68°C | 5.5 months | 29 |

From table 8 is evident that neither does the addition of a wetting agent lesson the enzyme activity from coupling yield point of view, which otherwise could be expected.

Table 8

|  | Enzyme activity in % of added enzyme activity |
|---|---|
| With Tween 80 | 57, 48, 45 |
| Without Tween 80 | 41, 44 |

Reported tests show that the addition of the wetting agent Tween 80 does not influence the enzyme paper in a negative way seen from an activity or sensitivity point of view, and at the same time the enzyme paper exhibits an almost perfect wettability and is soaked through within the course of a few seconds.

**Claims**

1. Enzyme paper for use in the indication of cholinesterase-inhibitors in gas or liquid, characterized in that it consists of an enzyme carrier of the type chromatography paper with ion exchange function and immobilized on the carrier a cholinesterase enzyme isolated from plaice, at least one the cholinesterase enzyme stabilizing substance, the stabilizing substance or substances being of a carbohydrate type, and as a wetting agent a surface-active agent.

2. Enzyme paper according to claim 1, characterized in that saccharose and dextran are used as the stabilizing substance.

3. Method of producing an enzyme paper according to claim 1 or 2, characterized in that a chromatography paper with ion exchange function, preferably a chromatography paper of DEAE-cellulose, is equilibrated with a sodiumchloride solution washed, dried and impregnated with a buffered solution comprising the enzyme cholinesterase from plaice, a stabilizing agent of the carbohydrate type, preferably saccharose and dextran, and a wetting agent, preferably Polysorbate 80, and then the paper is dried.

4. Use of the enzyme paper according to any one of claims 1-3 together with a substrate, which substrate is influenced by the enzyme with a colour change as the result, e. g. 2,6-dichloro indophenyl acetate or indoxyl acetate, for the indication in a gas or liquid, preferably in air or water, of a substance which inhibits the enzyme reaction.

5. Use according to claim 4, characterized in that the substrate is adsorbed on a porous carrier of paper, preferably of the type chromatography paper or filter paper.

6. Use according to claim 4 or 5, characterized in that the presence of a nerve gas or another cholinesterase-inhibitor in air or water is indicated by means of the enzyme paper and the substrate interacting therewith.

**Patentansprüche**

1. Enzympapier zum Nachweis von Cholinesterase-Inhibitoren in Gasen oder Flüssigkeiten, dadurch gekennzeichnet, daß es aus einem Enzymträger von der Art des Chromatographierpapiers mit Ionenaustauscherfunktion und einem auf dem Träger immobilisierten Cholinesteraseenzym, isoliert aus Schollen, wenigstens einer Stabilisierungssubstanz für Cholinesteraseenzym, wobei die Stabilisierungssubstanz oder -substanzen von der Art der Kohlenhydrate sind, und einem oberflächenaktiven Mittel als Benetzungsmittel besteht.

2. Enzympapier nach Anspruch 1, dadurch gekennzeichnet, daß als Stabilisierungssubstanz Saccharose und Dextran verwendet werden.

3. Verfahren zur Herstellung eines Enzympapiers nach Anspruch 1 oder 2, dadurch gekennzeichnet, daß man das Gleichgewicht eines Chromatographierpapiers mit Ionenaustauscherfunktion, vorzugsweise eines Papiers aus DEAE-Cellulose, mit einer Natriumchloridlösung einstellt, das Papier wäscht, trocknet und mit einer gepufferten Lösung enthaltend das Enzym Cholinesterase aus Scholle, ein Stabilisierungsmittel von der Art der Kohlenhydrate, vorzugsweise Saccharose und Dextran, und ein Benetzungsmittel, vorzugsweise Polysorbat 80, imprägniert, und das Papier sodann trocknet.

4. Verwendung des Enzympapiers nach einem der Ansprüche 1-3 zusammen mit einem Substrat, welches Substrat durch das Enzym mit einem Farbwechsel beeinflußt wird, wie z. B. 2,3-Dichlorindophenylacetat oder Indoxylacetat, beeinflußt wird, um in einem Gas oder einer Flüssigkeit, vorzugsweise in Luft oder Wasser, eine Substanz, welche die Enzymreaktion inhibiert, anzuzeigen.

5. Verwendung nach Anspruch 4, dadurch gekennzeichnet, daß das Substrat auf einem porösen Träger aus Papier, vorzugsweise Chromatographierpapier oder Filterpapier, adsorbiert ist.

6. Verwendung nach Anspruch 4 oder 5, dadurch gekennzeichnet, daß mittels des Enzympapiers und des damit zusammenwirkenden Substrats die Gegenwart eines Nervengases oder eines anderen Cholinesteraseinhibitors in Luft oder Wasser angezeigt wird.

**Revendications**

1. Papier enzymatique destiné à être utilisé pour l'indication d'inhibiteurs de cholinestérase dans un gaz ou un liquide, caractérisé en ce qu'il est constitué d'un support enzymatique du type d'un papier de chromatographie exerçant une fonction d'échange d'ions et, immobilisée sur le support, une enzyme cholinestérase isolée de plies, au moins une substance stabilisant l'enzyme cholinestérase, la ou les substances stabilisantes étant de type carbohydrate et, comme agent mouillant, un agent tensio-actif.

2. Papier enzymatique selon la revendication 1, caractérisé en ce que, comme substance stabilisante, on utilise le saccharose et le dextrane.

3. Procédé de formation d'un papier enzymatique selon la revendication 1 ou 2, caractérisé en ce qu'on équilibre un papier de chromatographie à fonction d'échange d'ions, de préférence un papier de chromatographie de diéthylamino-éthyl-cellulose, avec une solution de chlorure de sodium, on le lave, on le sèche et on l'imprègne d'une solution tamponnée comprenant l'enzyme cholinestérase de plies, un agent stabilisant de type carbohydrate, de préférence le saccharose et le dextrane, ainsi qu'un agent mouillant, de préférence le Polysorbate 80, après quoi on sèche le papier.

4. Utilisation du papier enzymatique selon l'une quelconque des revendications 1 à 3 conjointement avec un substrat, ce dernier étant influencé par l'enzyme avec, pour conséquence, un changement de coloration, par exemple, l'acétate de 2,6-dichloro-indophényle ou l'acétate d'indoxyle, afin d'indiquer la présence, dans un gaz ou un liquide, de préférence dans l'air ou dans l'eau, d'une substance inhibant la réaction enzymatique.

5. Utilisation selon la revendication 4, caractérisée en ce que le substrat est adsorbé sur un support de papier poreux, de préférence, un papier de type pour chromatographie ou un papier filtrant.

6. Utilisation selon la revendication 4 ou 5, caractérisée en ce que la présence d'un gaz neurotoxique ou d'un autre inhibiteur de cholinestérase dans l'air ou l'eau est indiquée au moyen du papier enzymatique et du substrat réagissant entre eux.